# EUROPÄISCHE PATENTSCHRIFT

(11) **EP 2 882 363 B1**
(45) Veröffentlichungstag und Bekanntmachung des Hinweises auf die Patenterteilung: **24.04.2019**
(21) Anmeldenummer: 13756297.1
(22) Anmeldetag: 26.07.2013
(51) Int. Cl.: A61B 18/14

(54) **VORRICHTUNG ZUR ENDOSKOPISCHEN SUBMUKOSADISSEKTION IM OBEREN ODER UNTEREN GASTROINTESTINALTRAKT**
DEVICE FOR ENDOSCOPIC SUBMUCOSAL DISSECTION IN THE UPPER OR LOWER GASTROINTESTINAL TRACT
DISPOSITIF POUR RÉALISER UNE DISSECTION ENDOSCOPIQUE SUBMUQUEUSE DANS LE TUBE DIGESTIF SUPÉRIEUR ET INFÉRIEUR

(30) Priorität: 12.08.2012 DE 102012015834
(43) Veröffentlichungstag der Anmeldung: 17.06.2015
(73) Patentinhaber: Medwork GmbH, 91315 Höchstadt/Aisch (DE); Assistance Publique Hôpitaux De Paris, 75004 Paris 4 (FR); UNIVERSITE PARIS DESCARTES, 75270 Paris Cedex 06 (FR)
(72) Erfinder: BERKHOUT, Barry, 91315 Höchstadt/Aisch (DE); FISCHER, Gerald, 91315 Höchstadt/Aisch (DE); STIRNWEISS, Matthias, 91475 Lonnerstadt (DE); BREHM, Andreas, 91325 Adelsdorf (DE); PRAT, Frédéric, F-92330 Sceaux (FR)
(74) Vertreter: Carstensen, Lars
(86) Internationale Anmeldenummer: PCT/DE2013/000413
(87) Internationale Veröffentlichungsnummer: WO 2014/026664

(56) Entgegenhaltungen:
- EP-A2- 1 849 426
- DE-A1- 3 220 940
- DE-A1- 3 419 962
- DE-A1- 3 626 371
- US-A- 3 903 892
- US-A- 6 007 546
- US-A1- 2004 172 018
- US-A1- 2010 036 375

## Beschreibung

Die Erfindung betrifft eine Vorrichtung zur endoskopischen Submukosadissektion im oberen oder unteren Gastrointestinaltrakt mit einem Tubus, der zumindest ein in dessen Längsrichtung verlaufendes Lumen aufweist, wobei durch das zumindest eine Lumen ein mit Hochfrequenzstrom bestrombarer Betätigungsdraht verläuft, an dessen distalem Ende ein als Messer wirkendes Hochfrequenzwerkzeug und an dessen proximalem Ende ein sich am Tubus abstützendes Betätigungselement angreift.

Gastrointestinale Frühneoplasien lassen sich mit Hilfe der endoskopischen Resektion beseitigen. Für die Resektion entsprechender Karzinome, die als Läsionen mit geringer Flächenausdehnung bis zu 2 cm oder als großflächige Läsionen mit Abmessungen von mehr als 2 cm auftreten können, werden unterschiedliche Vorrichtungen und Verfahren eingesetzt.

Bei kleinflächigen Läsionen kann das entsprechende Gewebe mittels einer endoskopischen Mukosaresektion (EMR), bei der es sich um eine Schlingenresektion oder Kappenmukosektomie handeln kann, abgetragen werden. Diese Vorrichtungen und Verfahren werden zwar zum Teil auch bei Läsionen mit größeren Abmessungen eingesetzt, wobei dann aber das Entfernen bzw. Abtragen der Läsion in mehreren Schritten erfolgen muss, d.h., die Läsion kann nicht en bloc abgetragen werden. Dabei sind jedoch das Blutungsrisiko auf Grund der größeren Anzahl von Schnitten und die Perforationsrate sehr hoch. Die in viele Einzelteile getrennte Läsion kann im Übrigen vom Pathologen kaum beurteilt werden, da er die Lateralränder nur sehr schwer identifizieren kann. Somit steigt auch das Risiko des Wiederauftretens von präkanzerösen Zellen erheblich.

Insbesondere bei großflächigen Läsionen wird daher seit einigen Jahren die endoskopische Submukosadissektion (ESD) angewandt, die eine Resektion großflächiger Frühkarzinome (>2 cm) en bloc ermöglicht. Dieses Verfahren, das mit der Vorrichtung gemäß der vorliegenden Erfindung durchgeführt werden soll, erfolgt endoskopisch in mehreren Arbeitsschritten.

Vorrichtungen zur endoskopischen Submukosadissektion im oberen oder unteren Gastrointestinaltrakt der im Oberbegriff des Patentanspruchs 1 genannten Gattung sind aus der Veröffentlichung der OLYMPUS MEDICAL SYSTEMS CORP., "Endoscopic Submucosal Dissection Product Catalog", Drucknummer: F0204E0-032012 bekannt.

In dieser Veröffentlichung werden mehrere Vorrichtungen beschrieben, die für die unterschiedlichen Arbeitsschritte einer endoskopischen Submukosadissektion verwendet werden. Es handelt sich dabei für die Arbeitsschritte umlaufende Inzision, Submukosadissektion und Hämostase um Vorrichtungen mit unterschiedlich ausgebildeten Messern, die als IT-Knife, Hook-Knife und Triangel-Tip-Knife bezeichnet werden. Das IT-Knife weist an seinem mit dem Betätigungsdraht verbundenen Elektrodenelement eine Keramikkugel auf. Diese soll die Spitze des Elektrodenelements isolieren und so verhindern, dass das Gewebe perforiert wird. Das Hook-Knife unterscheidet sich dadurch von der vorgenannten Vorrichtung, dass am distalen Ende ein L-förmiger Haken angeordnet ist, der ein Anheben der Mukosa für ein sicheres Schneiden ohne Perforation des darunterliegenden Gewebes ermöglichen soll. Das Flex-Knife weist eine ösenförmige Messerspitze auf, die über einen aus Einzelfasern gewundenen Draht mit dem Betätigungsdraht verbunden ist, wobei eine derart ausgebildete Messerspitze eine große Kontaktfläche zur Mukosa aufweisen soll. Dieses Flex-Knife soll gemäß der Druckschrift für jegliche Aufgaben von der Markierung der Läsion, über die Inzision, bis zur Dissektion bestens geeignet sein. Ein sogenanntes Dual-Knife mit einem pilzförmigen Ende lässt sich für die vorgenannten Arbeitsschritte in zwei unterschiedliche Positionen verstellen. Ein Triangel-Tip-Knife weist ein quer zum Betätigungsdraht verlaufendes dreieckiges Messer auf, das ein Schneiden in jede beliebige Richtung ermöglicht.

Weiterhin ist aus der DE 100 32 766 B4 ein endoskopisches Hochfrequenzmesser bekannt, bei dem ein Messerdraht zwischen zwei radialen Ausnehmungen des Tubus verläuft. Dabei ist der für eine Papillen-Sphincterektomie vorgesehene Messerdraht an seinem freien Ende über einen sogenannten Messerchip im Tubus festgelegt. Der Messerdraht verläuft folglich entlang eines Außenmantels des Tubus und lässt sich über ein proximal am Tubus vorgesehenes Betätigungselement derart verstellen, dass er sich mehr oder weniger bauchig über den Außenmantel des Tubus erstreckt. DE3626371 offenbart ein endoskopisches Instrument mit einer Drahtschlinge zur Polypektomie, US6007546 ein endoskopisches Instrument mit Drahtschlinge und Injektionsvorrichtung zur Submukosaresektion und Polypektomie.

Es ist Aufgabe der vorliegenden Erfindung, eine Vorrichtung der im Oberbegriff des Patentanspruchs 1 angegebenen Gattung derart weiterzubilden, dass diese für eine endoskopische Submukosadissektion, also auch zur Entfernung großflächiger Läsionen geeignet ist, wobei diese Vorrichtung für eine Durchführung sämtlicher Arbeitsschritte der endoskopischen Submukosadissektion geeignet sein soll, die jeweilige Vorrichtung zwischen den einzelnen Arbeitsschritten also nicht gegen eine andere ausgetauscht werden muss.

Diese Aufgabe wird, ausgehend vom Oberbegriff des Patentanspruchs 1, in Verbindung mit dessen kennzeichnenden Merkmalen gelöst.

Gemäß dem kennzeichnenden Teil des Patentanspruchs 1 soll das als Messer wirkende Hochfrequenzwerkzeug als Drahtlitze ausgebildet sein, die in zumindest drei Arbeitsstellungen verstellbar ist, wobei die Drahtlitze in einer maximal ausgefahrenen Stellung des distalen Endes des Betätigungsdrahtes eine Arbeitsstellung einnimmt, in der sie eine im wesentlichen sichelförmige Außenkontur aufweist, wobei ein mit dem Betätigungsdraht verbundener Abschnitt der Drahtlitze gegenüber deren mit einem freien Litzenende versehenen Abschnitt mit einem geringeren konkav verlaufenden Krümmungsradius ausgebildet ist und wobei die Drahtlitze zumindest im Bereich ihrer sichelförmigen konvex verlaufenden Außenkontur, sowohl nach innen als nach außen weisend, mit einer Schneide versehen ist.

Die Drahtlitze weist also insgesamt die Form einer asymmetrischen Schlinge auf, die aber als Schneideinrichtung genutzt wird. Dabei ist die Drahtlitze zu einer entsprechenden Messerspitze verformt, zu deren Stabilisierung ein Materialauftrag dienen kann. In der angegebenen Arbeitsstellung ermöglicht die Formgebung des sichelförmigen Messers, dass man sich mit der Messerspitze in Bindegewebsstränge einhakt und diese durch eine leichte Zugbewegung bei gleichzeitiger Hochfrequenz-Applikation durchtrennt. Der konvex ausgebildete Abschnitt der Drahtlitze kann ähnlich einer Sense verwendet werden, so dass durch Schwenkbewegungen der distalen Spitze des sichelförmigen Messers in größerem Umfang submuköse Areale durchtrennt werden.

Mit dem konvex ausgebildeten Schneidedraht lassen sich in vorteilhafter Weise ziehende Schnitte ausführen, so dass das Gewebe geschont wird.

Aus dieser Ausbildung der Drahtlitze als sichelförmiges Messer lässt diese sich durch eine Längsverstellung des Betätigungsdrahtes mittels des Betätigungselementes in weitere Arbeitsstellungen überführen, in denen im Rahmen der endoskopischen Submukosadissektion mittels dieser Vorrichtung bestimmte erforderliche oder zweckmäßige Arbeitsschritte durchgeführt werden können. Daher lässt sich die gleiche Vorrichtung durch eine entsprechende Veränderung der am distalen Ende der Vorrichtung vorgesehenen chirurgischen Einrichtung für die übrigen Arbeitsschritte verwenden, d. h., der Tubus mit samt der von seinem distalen Ende ausgehenden Drahtlitze verbleibt im Körper des Patienten und muss nicht gegen eine andere Vorrichtung ausgetauscht werden.

Weiterhin sollen gemäß dem Patentanspruch 2 die beiden Abschnitte am distalen Ende der Drahtlitze stoffschlüssig mit einander verbunden sein, wobei einer der beiden Abschnitte einen sich über die Spitze der Drahtlitze hinaus erstreckenden Fortsatz aufweist, der eine weitere Schneide bildet. Es besteht daher die Möglichkeit, für die Abschnitte der Drahtlitze unterschiedliche Drähte zu verwenden, so dass diese auf die mit ihnen durchzuführenden Arbeitsschritte abgestimmt werden können. Die Drahtlitze soll dabei auch im Bereich der stoffschlüssigen Verbindung der beiden Abschnitte, also an ihrer Spitze derart ausgebildet sein, dass die Schneideigenschaft erhalten bleibt.

Demgegenüber sind nach der gattungsbildenden Veröffentlichung "Endoscopic Submucosal Dissection Product Catalog" unterschiedliche endoskopische Vorrichtungen vorgesehen, die je nach dem durchzuführenden Arbeitsschritt und der dabei auftretenden Problematik gegeneinander auszutauschen sind. Diese Veröffentlichung zeigt im Übrigen kein Messer, das durch eine sichelförmig gestaltete Drahtlitze gebildet wird. Nach der DE 100 32 766 B4 ist zwar das Messer als mit einem Hochfrequenzstrom bestrombarer Messerdraht ausgebildet, dabei soll die endoskopische Vorrichtung aber vorzugsweise für eine Papillen-Sphincterekotomie vorgesehen sein. Wie bereits dargelegt, ist der Messerdraht in zwei in Längsrichtung des Tubus voneinander beabstandeten Ausnehmungen des Tubus geführt und kann durch eine Längsverstellung des Betätigungsdrahtes in eine Arbeitsstellung gebracht werden, in der der Messerdraht distal bauchig über den Tubus vorsteht. Es handelt sich demnach ebenfalls um kein Messer, das eine sichelförmige Außenkontur aufweist und um keine in unterschiedliche Arbeitsstellungen verstellbare Vorrichtung zur endoskopischen Chirurgie in einem oberen oder unteren Gastrointestinaltrakt.

Gemäß einer weiteren Ausgestaltung der Erfindung soll der einen geringeren Krümmungsradius aufweisende Abschnitt der Drahtlitze, der einen konvex verlaufenden Abschnitt der Außenkontur der Drahtlitze bildet, über einen gegenläufigen Radius oder einen geraden Abschnitt in eine Spitze des Messers und über einen gegenläufigen Radius in einen mit dem Betätigungsdraht verbundenen Abschnitt übergehen. Dadurch ergibt sich eine sichelförmige Außenschneide, die eine optimale Vorspannung aufweist, um Schneidevorgänge durchzuführen.

Weiterhin soll das Hochfrequenzwerkzeug gemäß der Erfindung zwei weitere Arbeitsstellungen aufweisen, wonach die Drahtlitze in einer im Wesentlichen halb ausgefahrenen Stellung des distalen Endes des Betätigungsdrahtes einen etwa haarnadelartigen Verlauf aufweist und in dessen eingezogener Stellung vollständig innerhalb des Lumens liegt. In der Arbeitsstellung, in der die Drahtlitze mit einer haarnadelartigen Außenkontur verläuft, kann diese Einrichtung um etwa 20° bis 30° zum axialen Verlauf des Tubus abgewinkelt sein, so dass die am gekrümmten Ende der haarnadelartig geformten Drahtlitze ausgebildeten Spitze durch vom Betätigungsdraht übertragene Drehbewegungen exakt positioniert werden kann. Die entsprechende Spitze, die etwa 1-3 mm aus dem Tubus vorstehen kann, dient dazu, zunächst Markierungen im Randbereich der Läsion vorzunehmen, wobei zu diesem Zweck die Spitze als Elektrode dient, mit der ein Hochfrequenzstrom auf die Mukosa übertragen wird.

Diese Markierungen sind zu Beginn der weiteren Behandlung sichtbar, da das Gewebe koaguliert wird, so dass das Wasser verdampft und sich an den Punkten eine bräunliche Verfärbung bildet. Mit dem haarnadelförmig geformten Messer kann im Anschluss daran ebenfalls mittels Hochfrequenzstromapplikation die Mukosa in Richtung Submukosa eröffnet werden. Daraus ergibt sich, dass die erfindungsgemäße Vorrichtung durch eine entsprechende Verstellung für die ersten im Rahmen einer endoskopischen Submukosadissektion vorzunehmenden Behandlung in entsprechender Weise einstellbar ist.

Weiterhin ist der Tubus an seinem distalen Ende zum Einstechen in die Mukosa ausgebildet und kann bestrombar sein, wobei das den Betätigungsdraht aufnehmende Lumen oder ein weiteres im Tubus verlaufendes Lumen als Injektionseinrichtung ausgebildet sind. Dabei nimmt das Hochfrequenzwerkzeug eine weitere Arbeitsstellung ein, in der die Drahtlitze allerdings vollständig in das Lumen eingezogen ist. Das distale Ende des Tubus wird in die geöffnete Stelle der Mukosa eingeführt, woraufhin eine Substanz unter die Läsion gespritzt wird, um die Mukosa von der Submukosa abzuheben. Durch das Unterspritzen der Mukosa entsteht ein Flüssigkeitspolster, so dass beim anschließenden Schnitt ein Sicherheitsabstand zum Muskelgewebe eingehalten werden kann. Anschließend wird die chirurgische Einrichtung in eine Arbeitsposition verstellt, die bereits im Zusammenhang mit dem Patentanspruch 1 erläutert wurde. Mittels des sichelförmigen, durch die Drahtlitze gebildeten Messers wird die Läsion zirkulär inzidiert und kann anschließend aufgrund der schlingenartigen Ausbildung des Messers en bloc abgetragen werden.

Im Zusammenhang mit diesen Verstellmöglichkeiten der Drahtlitze in unterschiedliche Arbeitspositionen wird weiterhin vorgeschlagen, dass das Hochfrequenzwerkzeug mit einer haptischen Signaleinrichtung verbunden ist, die das Erreichen der entsprechenden zuvor erläuterten Arbeitsstellungen als haptisches Signal an das Betätigungselement überträgt. Mittels dieser haptischen Signaleinrichtung wird also für den behandelnden Arzt, der mit einer Hand das Betätigungselement bedient, spürbar, wann die entsprechenden Arbeitspositionen erreicht sind, so dass jeweils eine präzise Einstellung der Vorrichtung erfolgen kann.

In diesem Zusammenhang ist weiterhin vorgesehen, dass die haptische Signaleinrichtung durch eine den Betätigungsdraht und das eine Ende der Drahtlitze aneinander fixierende Verbindungshülse, eine am freien Ende der Drahtlitze befestigten Mitnahmehülse, zu der der andere zum Befestigungsdraht führende Abschnitt der Drahtlitze frei beweglich ist, und eine am distalen Ende des Lumens im Tubus fixierten Hülse, die als axialer Anschlag für die Mitnahmehülse wirkt, gebildet wird. Durch die Anordnung der Hülse und der Mitnahmehülse zueinander innerhalb des Lumens und deren Zusammenwirken mit der Drahtlitze wird in der Mitte des Verstellbereiches des Betätigungsdrahtes eine spürbare Anschlagsposition geschaffen, die als haptisches Signal am Betätigungselement registriert werden kann.

Im Übrigen werden natürlich präzise Endpositionen für die völlig ausgefahrene Stellung der Drahtlitze, in der diese sichelförmig ausgebildet ist und die völlig eingefahrene Position der Drahtlitze geschaffen. In diesem Zusammenhang ist außerdem vorgesehen, dass die Drahtlitze in der Arbeitsstellung der Vorrichtung, in der sie das sichelförmige Messer bildet, mit ihrem einen Ende, ausgehend von dem gegenläufig gekrümmten Abschnitt, zur Längsmittelachse versetzt, an dem Inneren der Hülse und dem Inneren der Mitnahmehülse anliegt und anschließend durch eine Kröpfung in einen konzentrischen Verlauf übergeht. Dadurch ist die Drahtlitze in dieser Arbeitsstellung in vorteilhafter Weise mit den beiden sich in das Lumen erstreckenden Abschnitten an der Mitnahmehülse und an der im Tubus fixierten Hülse geführt.

Die vorstehend erläuterte Führung der Enden der Drahtlitze ermöglicht außerdem, dass die chirurgische Einrichtung in den Stellungen des Betätigungsdrahtes mit sichelartiger Außenkontur und mit haarnadelartiger Außenkontur der Drahtlitze gegenüber dem Tubus verdrehbar ist. Daher können sowohl bei der Markierung des Karzinoms als auch bei der anschließenden Dissektion erforderliche Bewegungen des Messers durchgeführt werden.

Weiterhin soll der Betätigungsdraht aus Nitinol hergestellt sein. Dadurch ergibt sich eine hohe Torsionssteifigkeit des Betätigungsdrahtes, so dass Drehbewegungen am proximalen Betätigungselement sehr präzise auf die distale Einrichtung übertragen werden und diese folglich sehr exakt positioniert und bewegt werden kann. Der Tubus ist vorzugsweise zumindest über Teilbereiche aus Teflon hergestellt.
Im Falle einer stoffschlüssigen Verbindung der beiden Abschnitte aneinander, die am distalen Ende der Drahtlitze erfolgt, kann als Verbindungstechnik Laserschweißen eingesetzt werden. Diese Verbindungstechnik ermöglicht eine Großserienfertigung der entsprechend geformten Drahtlitze.

Da die Drahtlitze aus zwei stoffschlüssig aneinander befestigten Drahtabschnitten besteht, können diese in weiterer Ausgestaltung der Erfindung aus unterschiedlichen Materialen und/oder aus Drahtabschnitten mit unterschiedlichen Querschnitten und/oder Querschnittsformen ausgebildet sein. Die Drahtabschnitte können beispielsweise wahlweise aus chirurgischem Edelstahl, Titan, einem Verbundwerkstoff (DFT) oder Wolfram bestehen. Die Querschnitte weisen wahlweise eine kreisrunde, ovale, rechteckige, dreieckige oder mehreckige Geometrie auf. Bei dem Draht kann es sich um einen monofilen oder geflochtenen Draht handeln. Der Draht kann auch gehämmert sein, so dass dessen Oberfläche verdichtet ist.

In weiterer Ausgestaltung der Erfindung soll einer der beiden Abschnitte einen sich über die Spitze der Drahtlitze hinaus erstreckenden Fortsatz aufweisen, der eine weitere Schneide bildet. Vorzugsweise weist dabei der Abschnitt, der in der ersten Stellung der Drahtlitze mit einem geringeren konkav verlaufenden Krümmungsradius ausgebildet ist, einen sich über die Spitze der Drahtlitze erstreckenden Fortsatz auf. Der Fortsatz kann etwa 1 bis 2 mm, vorzugsweise 1,5 mm, über die Spitze vorstehen. Dieses nadelartig ausgebildete Messer soll vorzugsweise zum Einschneiden in das Gewebe verwendet werden, wobei aufgrund der nadelartigen Ausbildung des Messers entsprechende Schnitte mit hoher Zielgenauigkeit und mit einer definierten Tiefe ausgeführt werden können. Ein zirkuläres Einschneiden der Mukosa kann beispielsweise mit einer Stellung der Vorrichtung durchgeführt werden, in welcher die Drahtlitze soweit in das Lumen eingezogen ist, dass nur noch der nadelartige Fortsatz über das distale Ende des Tubus' vorsteht. Dabei lässt sich die Incisionstiefe stufenlos einstellen. Der Fortsatz kann entweder einen geraden oder einen gekrümmten Verlauf aufweisen.

Schließlich bezieht sich die Offenbarung auch auf ein Verfahren zur endoskopischen Submukosadissektion eines transmukosal begrenzten Karzinoms oder eines gut differenzierten Adenokarzinoms >20 mm im oberen und unteren Gastrointestinaltrakt mittels eines über einen Betätigungsdraht bewegbaren und mit einem Hochfrequenzstrom beaufschlagbaren Messers, wobei der Betätigungsdraht das Messer an seinem distalen Ende aufnimmt, über ein Lumen in einem Tubus geführt ist und über ein am proximalen Ende des Tubus angeordneten Betätigungselement verstellt wird. Dabei soll erfindungsgemäß das durch eine Drahtlitze gebildete Messer durch eine Längsverschiebung des Betätigungsdrahtes in eine erste Arbeitsstellung verstellt werden, die im Wesentlichen einer Mittelstellung des Betätigungselements entspricht. In dieser soll das Messer eine haarnadelförmige Außenkontur aufweisen, wobei mit seiner bestromten Spitze um das Karzinom herum Markierungen gesetzt werden, woraufhin das Messer in dieser Arbeitsposition ebenfalls zum Eröffnen der Mukosa in Richtung der Submukosa verwendet wird. Anschließend wird die Drahtlitze durch eine Längsverschiebung des Betätigungsdrahtes in das Lumen eingezogen, wobei das Betätigungselement eine erste Endstellung einnimmt, woraufhin mit dem Ende des Tubus vom Randbereich des Karzinoms her in die Mukosa eingestochen und das Karzinom mit einer über das Lumen zugeführten flüssigen Substanz unterspritzt und von der Submukosa abgehoben wird. Über das Lumen kann auch eine Spülflüssigkeit in Richtung des distalen Endes der Vorrichtung geleitet werden, mit welcher das Gewebe gespült und/oder das Messer gereinigt wird. Dadurch lassen sich zum einen die Sicht und zum anderen die Schnittqualität verbessern. Anschließend wird die Drahtlitze in eine dritte Arbeitsstellung überführt, in der das Betätigungselement seine zweite Endstellung einnimmt. In dieser Arbeitsstellung des Messers wird das Karzinom mit den äußeren und inneren Schneiden des sichelförmigen Messers inzidiert und über die durch die Drahtlitze gebildete Schlinge abgetragen. Daher kann die gesamte Submukosadissektion mittels einer entsprechenden Vorrichtung und eines mit entsprechenden Arbeitsschritten ausgeführten Verfahrens durchgeführt werden. Folglich kann die Dauer des endoskopisch durchgeführten Eingriffs gegenüber den mit bekannten Vorrichtungen durchgeführten Eingriffen erheblich reduziert werden.

Die Erfindung ist nicht auf die angegebene Kombination der Merkmale des unabhängigen Patentanspruchs und der abhängigen Patentansprüche beschränkt. Es ergeben sich darüber hinaus weitere Möglichkeiten, einzelne Merkmale, insbesondere dann, wenn sie sich aus den Patentansprüchen, den zu diesen angegebenen Vorteilen, der nachfolgenden Beschreibung der Ausführungsbeispiele oder unmittelbar aus der Zeichnung ergeben, miteinander zu kombinieren. Außerdem soll die Bezugnahme der Patentansprüche auf die Zeichnung durch die Verwendung von Bezugszeichen den Schutzumfang der Patentansprüche auf keinen Fall auf die dargestellten Ausgestaltungsbeispiele beschränken.

Weitere Merkmale der Erfindung ergeben sich im Übrigen aus der nachfolgenden Beschreibung und aus der Zeichnung, in der zwei Ausführungsbeispiele der Erfindung vereinfacht dargestellt ist.

Es zeigen:
- Figur 1: eine Seitenansicht einer erfindungsgemäßen Vorrichtung zur endoskopischen Resektion mit einem sichelförmigen Messer,
- Figur 2: eine vergrößerte Darstellung des distalen Endes der Vorrichtung nach Figur 1 gemäß einem Ausschnitt II in Figur 1,
- Figur 3: das distale Ende einer entsprechenden Vorrichtung, wobei das Hochfrequenzwerkzeug eine Arbeitsstellung einnimmt, in der die entsprechende Drahtlitze einen haarnadelartigen Verlauf aufweist,
- Figur 4: ein distales Ende der Vorrichtung, bei welchem sich die Einrichtung in einer Arbeitsstellung befindet, in der die Drahtlitze vollständig in das Lumen eingezogen ist in einem gegenüber den Figuren 2 und 3 verkleinerten Maßstab,
- Figur 5: im Längsschnitt, ein distales Ende einer weiteren Ausbildung der Vorrichtung, bei welcher eine Drahtlitze mit einem sich über deren Spitze hinaus erstreckenden Fortsatz versehen ist,
- Figur 6: im Längsschnitt, eine gemäß Figur 5 ausgebildete Drahtlitze, die sich in einem halb ausgefahrenen Zustand befindet, und
- Figur 7: im Längsschnitt, eine gemäß Figur 5 ausgebildete Drahtlitze, die so weit in ein Lumen eingezogen ist, dass nur noch der Fortsatz über ein distales Ende des Tubus' vorsteht.

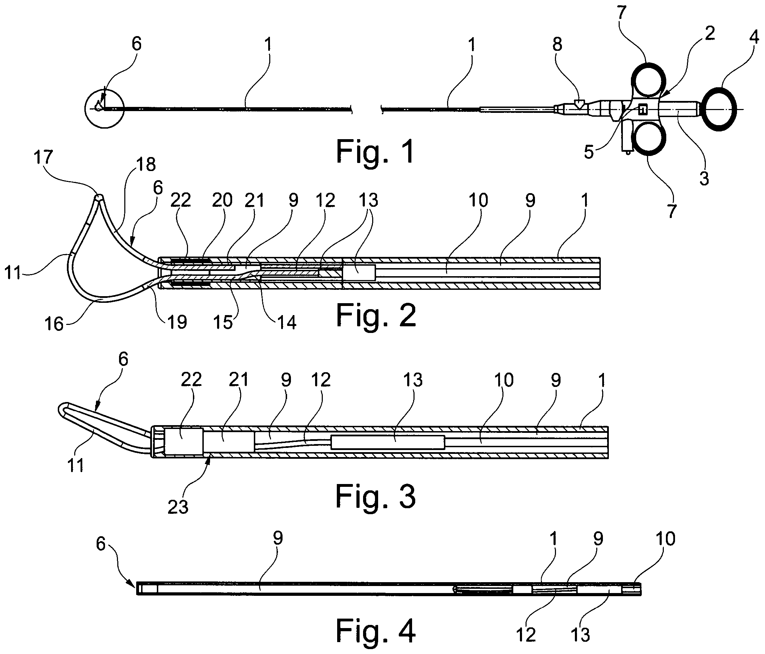

In der Figur 1 ist mit 1 ein verkürzt dargestellter Tubus bezeichnet, an dessen proximalem Ende ein Betätigungselement 2 angeordnet ist. Dieses Betätigungselement 2 weist einen am Tubus 1 über ein Führungselement 3 abgestützten Daumenring 4 auf, wobei auf dem Führungselement eine Führungsbuchse 5 längsverschiebbar geführt ist, die eine Verstellung eines am distalen Ende des Tubus 1 angeordneten Hochfrequenzwerkzeuges 6 bewirkt. An der Führungsbuchse sind dabei Fingerringe 7 angeordnet, so dass der behandelnde Arzt, mit einer Hand eine Verstellung des Hochfrequenzwerkzeuges 6 durchführen kann. Außerdem ist an der Betätigungseinrichtung 2 ein Stutzen 8 vorgesehen, über den in den Tubus 1 eine flüssige Substanz eingeleitet werden kann, die, wie noch weiter erläutert werden wird, bis an das distale Ende der Vorrichtung geführt wird. Wie weiterhin der Figur 1 entnommen werden kann, weist das Hochfrequenzwerkzeug 6 eine sichelförmige Außenkontur auf.

Die Darstellung nach der Figur 2 zeigt einen vergrößerten Ausschnitt des distalen Endes der Anordnung nach der Figur 1, wobei in der Schnittdarstellung, soweit diese aus der linken Bildhälfte hervorgeht, einen Schnitt durch sämtliche Bauelemente darstellt, während in der rechten Bildhälfte nur ein Schnitt durch den Tubus 1 erfolgt ist. Danach weist der Tubus 1 in seinem Inneren ein Lumen 9 auf, durch welches ein aus Nitinol hergestellter Betätigungsdraht 10 verläuft. Weiterhin nimmt das Lumen 9 am distalen Ende teilweise eine Drahtlitze 11 auf, die sich in der Figur 2 in einem ausgefahrenen Zustand befindet, in welchem sie ihre distale Endposition einnimmt und eine sichelartige Außenkontur aufweist. Die Drahtlitze 11 ist mit einem ersten Ende 12 über eine Verbindungshülse 13 mit dem Betätigungsdraht 10 verbunden. Diese Verbindungshülse 13 ist dabei gleitend im Lumen 9 geführt, wobei sie allerdings gegenüber einer Innenmantelfläche des Lumens 9 ein derartiges Spiel aufweist, dass eine über den Stutzen zugeführte flüssige Substanz bis an das distale Ende des Tubus 1 gelangen kann. Zu diesem Zweck kann die Verbindungshülse 13 auch mit Nuten versehen sein, über die die flüssige Substanz in axialer Richtung zwischen der Verbindungshülse 13 und dem Mantel des Lumens 9 strömen kann.

Wie weiterhin aus der Figur 2 hervorgeht, ist das erste Ende 12 der Drahtlitze 11 mit einem koaxialen Verlauf zum Tubus 1 ausgeführt, soweit es sich innerhalb der Verbindunghülse 13 erstreckt. Daran anschließend weist die Drahtlitze 11 eine Kröpfung 14 auf, an die sich ein im Wesentlichen gerader, im Wandbereich des Tubus verlaufender Abschnitt 15 anschließt. Die Drahtlitze 11, die eine sichelförmige Außenkontur aufweist, also sichelförmig verläuft, besteht weiterhin aus einem konvex gekrümmten Abschnitt 16, einer Spitze 17 und einem konkav gekrümmten Abschnitt 18. Wie die Figur 2 weiterhin verdeutlicht, verläuft die Drahtlitze 11 im Bereich zwischen der Spitze 17 und dem konvexen Abschnitt 16 im Wesentlichen gerade oder mit leicht gegenläufiger Krümmung. Der konvex gekrümmte Abschnitt 16 geht im Übrigen über einen gegenläufig gekrümmten Abschnitt 19 in den wandnahen Abschnitt 15 über. Außerdem geht von dem konkav gekrümmten Abschnitt 18 ein freies Ende 20 der Drahtlitze 11 aus, das an einer Mitnahmehülse 21 befestigt ist.

Diese Mitnahmehülse 21 liegt gemäß der Figur 2 stirnseitig an der Verbindungshülse 13 an und ist in einer am distalen Ende des Lumens 9 fixierten Hülse 22 geführt. Ferner ist das freie Ende 20 in der Mitnahmehülse 21 fixiert, so dass die Mitnahmehülse 21 den Bewegungen des freien Endes 20 folgt und dabei gegenüber dem wandnahen Abschnitt 15 der Drahtlitze 11 verschoben wird. Die gesamte vorstehend beschriebene Anordnung dient als in der Figur 3 mit 23 bezeichnete haptische Signaleinrichtung deren Funktion im Weiteren noch erläutert werden wird.

In der Figur 3 ist wiederum im Rahmen eines Längsschnittes durch den Tubus 1 die Anordnung von Betätigungsdraht 10, Verbindungshülse 13, Drahtlitze 11, Mitnahmehülse 21 und Hülse 22 dargestellt, allerdings in einem gegenüber den Figuren 2 und 3 verkleinerten Maßstab. Dabei befindet sich die Drahtlitze 11 in einem halb ausgefahrenen Zustand, in dem die durch die Drahtlitze 11 gebildete chirurgische Einrichtung 6 eine haarnadelförmige Außenkontur aufweist. Dabei ist das haarnadelförmige Element um einen Winkel von 20° bis 30° gegenüber einer Längsachse des Tubus 1 abgewinkelt.

Gemäß der Figur 4 befindet sich die chirurgische Einrichtung 6 in einem Zustand, in dem die als Messer dienende Drahtlitze 11 vollständig in das Lumen 9 eingezogen ist.

Nachfolgend wird kurz die Verstellung der Drahtlitze 10 gegenüber dem Tubus 1 zur Erreichung unterschiedlicher Arbeitspositionen der chirurgischen Einrichtung 6 beschrieben:
In dem in der Figur 4 dargestellten Zustand befindet sich die mit den Fingerringen 7 versehene Führungsbuchse 5 in einem komplett eingefahrenen Zustand, d. h., die Führungsbuchse 5 befindet sind in einer unmittelbar dem Daumenring 4 benachbarten Stellung. Das führt dazu, dass die Drahtlitze 11 in gestreckter Form im Lumen 9 liegt. Die zuvor beschriebenen Bauelemente Hülse 22, Mitnahmehülse 21 und Verbindungshülse 13 liegen separiert voneinander in einem definierten Abstand in ihren jeweiligen Ausgangspositionen.

Wird nun die Führungsbuchse 5 über die Fingerringe 7 in Richtung des distalen Endes bewegt, so führt das dazu, dass die Mitnahmehülse 21 bis an die fest im Tubus 1 angeordnete Hülse 22 verschoben wird, so dass deren beiden Stirnseiten aneinanderstoßen. Dabei fährt die Drahtlitze 11 so weit aus dem distalen Ende des Tubus 1 aus, dass sich an der Drahtlitze 11 eine haarnadelartige Form ausbildet. Durch den Anschlag der Mitnahmehülse 21 an der feststehenden Hülse 22 ergibt sich ein Widerstand, der vom Anwender am Betätigungselement 2 als haptisches Signal empfunden wird. Durch die Reibung der Drahtlitze an der Innenmantelfläche des Lumen 9 sowie an den Innenmantelflächen der Mitnahmehülse 21 und der Hülse 22 ist die Führungsbuchse 5 in dieser Position arretiert. Dabei besteht weiterhin ein Abstand zwischen der Mitnahmehülse 21 und der Verbindungshülse 13.

Zum Erreichen eines ausgefahrenen Zustands wird die Führungsbuchse 5, die mit dem Betätigungsdraht 10 verbunden ist, weiter in distaler Richtung verschoben, nachdem der Widerstand zwischen der Drahtlitze 11 einerseits und der Mitnahmehülse 21 sowie der Hülse 22 überwunden ist. Diese Bewegung führt dazu, dass die Drahtlitze 11 komplett aus dem Tubus 1 ausfährt und sich an der Drahtlitze 11 eine Sichelform ausbildet. Diese Bewegung erfolgt so lange, bis die Verbindungshülse 13 gegen die Mitnahmehülse 21 stößt. Durch das Ausbauchen der Drahtlitze 11 ist diese in der ausgefahrenen Stellung arretiert. Der Widerstand, der zwischen der ausgebauchten Drahtlitze 11 sowie dem Lumen 9 und den Hülsen 21 und 22 herrscht, reicht aus um die Drahtlitze 11 in der gewünschten Sichelform zu halten.

Den vorstehenden Erläuterungen kann entnommen werden, dass sich mit der in ihrer Form verstellbaren Drahtlitze 11 Schneiden schaffen lassen, die für den jeweiligen Arbeitsschritt optimal gestaltet sind. Die erfindungsgemäße Vorrichtung ist im Rahmen einer endoskopischen Submukosadissektion universell einsetzbar, d.h., über das proximale Betätigungselement kann die Gestalt der chirurgischen Einrichtung entsprechend den jeweiligen Anforderungen geändert werden.

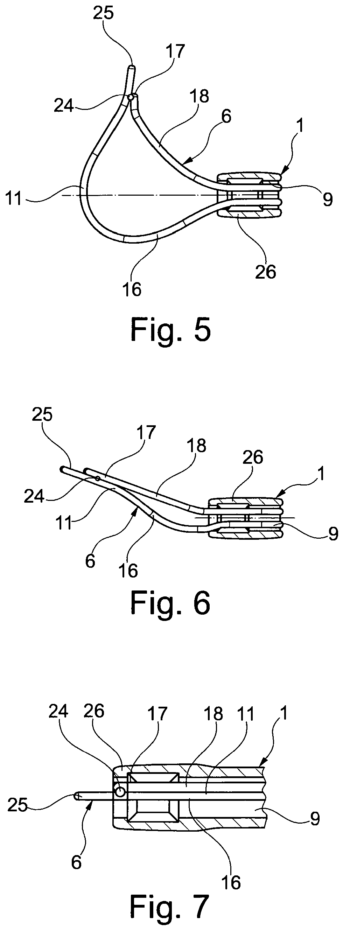

Aus den Figuren 5 bis 7 geht ein weiteres Ausgestaltungsbeispiel der Erfindung hervor. Bei diesem sind, wie insbesondere aus der Darstellung nach der Figur 5 ersichtlich ist, die beiden Abschnitte 16 und 18 der Drahtlitze 11 in dessen Spitze 17 stoffschlüssig über eine Laserschweißung 24 miteinander verbunden. Von dem Abschnitt 16 aus erstreckt sich ein Fortsatz 25, der über die Spitze 17 der Drahtlitze 11 hinausragt. Der jeweilige Verlauf dieses Fortsatzes 25 kann im Einzelnen den Figuren 5 bis 7 entnommen werden.

In der Figur 7 befindet sich die Drahtlitze 11 im Wesentlichen innerhalb des Lumens 9. Nur der Fortsatz 25 steht über ein distales Ende 26 des Tubus' 1 vor und kann als nadelartig ausgebildetes Messer benutzt werden. Mittels dieses nadelartigen Messers lassen sich äußerst präzise Schnitte mit definierter Tiefe ausführen. Bei diesen Schnitten kann es sich beispielsweise um ein zirkuläres Einschneiden der Mukosa handeln.

Da die Abschnitte 16 und 18 an der Spitze 17 der Drahtlitze 11 stoffschlüssig miteinander verbunden sind, können die jeweilige Ausbildung, der Werkstoff und die Abmessungen der beiden Abschnitte 16 und 18 voneinander abweichen. So kann beispielsweise der Querschnitt des Abschnitts 16 anders gestaltet sein als der des Abschnitts 18. Außerdem kann sich auch die Materialeigenschaft des Fortsatzes 25 von den Materialeigenschaften der beiden Abschnitte 16 und 18 unterscheiden.

### Bezugszeichenliste

- 1: Tubus
- 2: Betätigungselement
- 3: Führungselement
- 4: Daumenring
- 5: Führungsbuchse
- 6: Hochfrequenzwerkzeug
- 7: Fingerringe
- 8: Stutzen
- 9: Lumen
- 10: Betätigungsdraht
- 11: Drahtlitze
- 12: Erstes Ende von 11
- 13: Verbindungshülse zwischen 10 und 11
- 14: Kröpfung von 11
- 15: Wandnaher Abschnitt von 11
- 16: Konvex gekrümmter Abschnitt von 11
- 17: Spitze von 11
- 18: Konkav gekrümmter Abschnitt von 11
- 19: Gegenläufig gekrümmter Abschnitt von 11
- 20: Freies Ende von 11
- 21: Mitnahmehülse
- 22: Hülse in 9
- 23: Haptische Signaleinrichtung
- 24: Laserschweißung
- 25: Fortsatz
- 26: distales Ende von 1

## Patentansprüche

1. Vorrichtung zur endoskopischen Submukosadissektion im oberen oder unteren Gastrointestinaltrakt mit einem Tubus (1), der zumindest ein in dessen Längsrichtung verlaufendes Lumen (9) aufweist, wobei durch das zumindest eine Lumen (9) ein mit Hochfrequenzstrom bestrombarer Betätigungsdraht (10) verläuft, an dessen distalem Ende ein als Messer wirkendes Hochfrequenzwerkzeug (6) und an dessen proximalem Ende ein sich am Tubus (1) abstützendes Betätigungselement (2) angreift, **dadurch gekennzeichnet, dass** das als Messer wirkende Hochfrequenzwerkzeug (6) als Drahtlitze (11) ausgebildet ist, die in zumindest drei Arbeitsstellungen verstellbar ist, wobei die Drahtlitze (11) in einer ersten maximal ausgefahrenen Stellung des distalen Endes des Betätigungsdrahtes (10) eine Arbeitsstellung einnimmt, in der sie eine im Wesentlichen sichelförmige Außenkontur aufweist, dass ein mit dem Betätigungsdraht (10) verbundener Abschnitt (16) der Drahtlitze (11) gegenüber deren mit einem freien Ende (20) versehenen Abschnitt (18) mit einem geringeren konkav verlaufenden Krümmungsradius ausgebildet ist, dass die Drahtlitze (11) zumindest im Bereich ihrer sichelförmigen konvex verlaufenden Außenkontur (16), sowohl nach innen als nach außen weisend, mit einer Schneide versehen ist und in zwei weitere Arbeitsstellungen verstellbar ist, wonach die Drahtlitze (11) in einer zweiten Arbeitsstellung in einer im Wesentlichen halb ausgefahrenen Stellung des distalen Endes des Betätigungsdrahtes (10) einen etwa haarnadelartigen Verlauf aufweist und in einer dritten Arbeitsstellung in einer eingezogene Stellung des Betätigungsdrahtes (10) vollständig innerhalb des Lumens (9) liegt, und dass der Tubus (1) an seinem distalen Ende zum Einstechen in die Mukosa ausgebildet ist, wobei das den Betätigungsdraht (10) aufnehmende Lumen (9) oder ein weiteres im Tubus (1) verlaufendes Lumen als Injektionseinrichtung ausgebildet sind.

2. Vorrichtung nach Anspruch 1, **dadurch gekennzeichnet, dass** die beiden Abschnitte (16 und 18) am distalen Ende der Drahtlitze (11) stoffschlüssig miteinander verbunden sind und einer der beiden Abschnitte (16 oder 18) einen sich über die Spitze (17) der Drahtlitze (11) hinaus erstreckenden Fortsatz aufweist, der eine weitere Schneide bildet.

3. Vorrichtung nach einem der Patentansprüche 1 oder 2, **dadurch gekennzeichnet, dass** der einen geringeren Krümmungsradius aufweisende Abschnitt (16) der Drahtlitze (11), der konvex ausgebildet ist, über einen gegenläufigen Radius oder einen geraden Abschnitt (19) in eine Spitze (17) des Messers und über einen gegenläufigen Radius in den mit dem Betätigungsdraht verbundenen Abschnitt übergeht.

4. Vorrichtung nach Patentanspruch 1 oder 2, **dadurch gekennzeichnet, dass** der Tubus (1) an seinem distalen Ende zum Einstechen in die Mukosa bestrombar ist.

5. Vorrichtung nach Patentanspruch 1 oder 2, **dadurch gekennzeichnet, dass** das Hochfrequenzwerkzeug (6) mit einer haptischen Signaleinrichtung (23) verbunden ist, die das Erreichen der Funktionsstellungen als haptisches Signal an das Betätigungselement (2) überträgt.

6. Vorrichtung nach Patentanspruch 5, **dadurch gekennzeichnet, dass** die haptische Signaleinrichtung (23) durch eine den Betätigungsdraht (10) und das eine Ende der Drahtlitze (11) aneinander fixierende Verbindungshülse (13), eine am freien Ende (20) der Drahtlitze (11) befestigten Mitnahmehülse (21), zu der der andere zum Betätigungsdraht (10) führende Abschnitt der Drahtlitze (11) frei beweglich ist, und eine am distalen Ende des Lumens (9) im Tubus (1) fixierten Hülse (22), die als axialer Anschlag für die Mitnahmehülse (21) wirkt, gebildet wird.

7. Vorrichtung nach Patentanspruch 1 oder 2, **dadurch gekennzeichnet, dass** die Drahtlitze (11) in der Arbeitsstellung der Vorrichtung, in der sie das sichelförmige Messer bildet, mit ihrem einen Ende, ausgehend von dem gegenläufig gekrümmten Abschnitt, zur Längsmittelachse versetzt an dem Inneren der Hülse (22) und dem Inneren der Mitnahmehülse (21) anliegt und anschließend durch eine Kröpfung (14) in einen konzentrischen Verlauf übergeht.

8. Vorrichtung nach Patentanspruch 1 oder 2, **dadurch gekennzeichnet, dass** das Hochfrequenzwerkzeug (6) in den Stellungen des Betätigungsdrahtes (10) mit sichelartiger Außenkontur und mit haarnadelartiger Außenkontur der Drahtlitze (11) verdrehbar ist.

9. Vorrichtung nach einem der Patentansprüche 1 oder 2, **dadurch gekennzeichnet, dass** der Betätigungsdraht (10) aus Nitinol hergestellt ist.

10. Vorrichtung nach einem der Patentansprüche 2 bis 9, **dadurch gekennzeichnet, dass** die beiden Abschnitte (16 und 18) am distalen Ende der Drahtlitze (11) durch Laserschweißung aneinander befestigt sind.

11. Vorrichtung nach einem der Patentansprüche 2 bis 10, **dadurch gekennzeichnet, dass** die beiden Abschnitte (16 und 18) aus unterschiedlichen Materialen hergestellt sind.

12. Vorrichtung nach einem der Patentansprüche 2 bis 11, **dadurch gekennzeichnet, dass** die beiden Abschnitte (16 und 18) mit unterschiedlichen Querschnitten und/oder Querschnittsformen ausgebildet sind.

13. Vorrichtung nach einem der Patentansprüche 2 bis 12, **dadurch gekennzeichnet, dass** die Vorrichtung zum zirkulären Einschneiden der Mukosa eine weitere Stellung einnimmt, in welcher die Drahtlitze (11) soweit in das Lumen (9) eingezogen ist, dass nur noch der nadelartige Fortsatz (25) über das distale Ende des Tubus' (1) vorsteht.

14. Vorrichtung nach einem der Patentansprüche 2 bis 13, **dadurch gekennzeichnet, dass** der Fortsatz einen geraden Verlauf aufweist.

15. Vorrichtung nach einem der Patentansprüche 2 bis 13, **dadurch gekennzeichnet, dass** der Fortsatz einen gekrümmten Verlauf aufweist.

## Claims

1. Apparatus for endoscopic submucosal dissection in the upper or lower gastrointestinal tract, comprising a tube (1), which has at least one lumen (9) running in the longitudinal direction thereof, wherein an actuating wire (10), which can be energized by high-frequency current, runs through the at least one lumen (9), a high-frequency tool (6) acting as a knife engages the distal end of said wire and an actuating element (2) supported on the tube (1) engages the proximal end of said wire, **characterized in that** the high-frequency tool (6) acting as a knife is formed as a stranded wire (11), which is adjustable into at least three working positions, wherein, in a first maximally extended position of the distal end of the actuating wire (10), the stranded wire (11) assumes a working position in which it has a substantially crescent-shaped outer contour, **in that** a portion (16) of the stranded wire (11) that is connected to the actuating wire (10) is formed with a smaller radius of a concavely running curvature in comparison with a portion (18) thereof that is provided with a free end (20), **in that**, at least in the region of its crescent-shaped convexly running outer contour (16), the stranded wire (11) is provided with a cutting edge, facing both inward and outward and is adjustable into two further working positions, whereby the stranded wire (11) in a second working position follows an approximately hairpin-like path in a substantially half-extended position of the distal end of the actuating wire (10) and in a third working position lies completely within the lumen (9) in a retracted position of the actuating wire (10), and **in that** the tube (1) is formed at its distal end for piercing into the mucosa, wherein the lumen (9) receiving the actuating wire (10) or a further lumen running in the tube (1) is formed as an injection device.

2. Apparatus according to Claim 1, **characterized in that** the two portions (16 and 18) are connected to one another with a material bond at the distal end of the stranded wire (11) and one of the two portions (16 or 18) has a continuation that extends beyond the tip (17) of the stranded wire (11) and forms a further cutting edge.

3. Apparatus according to either of Patent Claims 1 and 2, **characterized in that** the portion (16) of the stranded wire (11) that has a smaller radius of curvature and is convexly formed goes over via an opposing radius or a straight portion (19) into a tip (17) of the knife and via an opposing radius into the portion that is connected to the actuating wire.

4. Apparatus according to Patent Claim 1 or 2, **characterized in that** tube (1) can be energized at its distal end for insertion into the mucosa.

5. Apparatus according to Patent Claim 1 or 2, **characterized in that** the high-frequency tool (6) is connected to a haptic signaling device (23), which transmits the reaching of the functional positions to the actuating element (2) as a haptic signal.

6. Apparatus according to Patent Claim 5, **characterized in that** the haptic signaling device (23) is formed by a connecting sleeve (13), which fixes the actuating wire (10) and one end of the stranded wire (11) to one another, a driving sleeve (21), which is fastened to the free end (20) of the stranded wire (11) and in relation to which the other portion of the stranded wire (11), leading to the actuating wire (10), is freely movable, and a sleeve (22), which is fixed to the distal end of the lumen (9) in the tube (1) and acts as an axial stop for the driving sleeve (21).

7. Apparatus according to Patent Claim 1 or 2, **characterized in that**, in the working position of the apparatus in which it forms the crescent-shaped knife, the stranded wire (11) lies with its one end, extending from the oppositely curved portion, against the interior of the sleeve (22) and the interior of the driving sleeve (21), while being offset from the longitudinal center axis, and subsequently goes over into a concentric path by following a crank (14).

8. Apparatus according to Patent Claim 1 or 2, **characterized in that** the high-frequency tool (6) is rotatable in the positions of the actuating wire (10) with a crescent-shaped outer contour and with a hairpin-like outer contour of the stranded wire (11).

9. Apparatus according to either of Patent Claims 1 and 2, **characterized in that** the actuating wire (10) is produced from nitinol.

10. Apparatus according to one of Patent Claims 2 to 9, **characterized in that** the two portions (16 and 18) are fastened to one another at the distal end of the stranded wire (11) by laser welding.

11. Apparatus according to one of Patent Claims 2 to 10, **characterized in that** the two portions (16 and 18) are produced from different materials.

12. Apparatus according to one of Patent Claims 2 to 11, **characterized in that** the two portions (16 and 18) are formed with different cross sections and/or cross-sectional shapes.

13. Apparatus according to one of Patent Claims 2 to 12, **characterized in that**, for circular cutting into the mucosa, the apparatus assumes a further position in which the stranded wire (11) is retracted into the lumen (9) to such an extent that only the needle-like continuation (25) projects beyond the distal end of the tube (1).

14. Apparatus according to one of Patent Claims 2 to 13, **characterized in that** the continuation follows a straight path.

15. Apparatus according to one of Patent Claims 2 to 13, **characterized in that** the continuation follows a curved path.

## Revendications

1. Dispositif pour réaliser une dissection endoscopique sous-muqueuse dans le tube digestif supérieur ou inférieur avec un tube (1), qui présente au moins une lumière (9) s'étendant dans sa direction longitudinale, dans lequel un fil d'actionnement (10) pouvant être alimenté par un courant à haute fréquence s'étend à travers ladite au moins une lumière (9), à l'extrémité distale duquel un outil à haute fréquence (6) agissant comme couteau est attaché et à l'extrémité proximale duquel un élément d'actionnement (2) s'appuyant sur le tube (1) est attaché, **caractérisé en ce que** l'outil à haute fréquence agissant comme couteau (6) est réalisé sous forme de toron (11), qui peut être déplacé dans au moins trois positions de travail, dans lequel le toron (11) occupe dans une position étendue au maximum de l'extrémité distale du fil d'actionnement (10) une position de travail dans laquelle il présente un contour extérieur essentiellement en forme de croissant, **en ce qu'**une partie (16) du toron (11) reliée au fil d'actionnement (10) est réalisée par rapport à sa partie (18) dotée d'une extrémité libre (20) avec un plus petit rayon de courbure en forme concave, **en ce que** le toron (11) est doté d'un tranchant au moins dans la région de son contour extérieur convexe en forme de croissant (16), tourné aussi bien vers l'intérieur que vers l'extérieur, et peut être déplacé dans deux autres positions de travail, selon lesquelles le toron (11) présente dans une deuxième position de travail une allure sensiblement en épingle à cheveux dans une position essentiellement à moitié étendue de l'extrémité distale du fil d'actionnement (10) et est situé dans une troisième position de travail dans une position rentrée du fil d'actionnement (10) entièrement à l'intérieur de la lumière (9), et **en ce que** le tube (1) est réalisé à son extrémité distale de façon à pénétrer dans la muqueuse, dans lequel la lumière (9) contenant le fil d'actionnement (10) ou une autre lumière s'étendant dans le tube (1) est réalisée sous forme de dispositif d'injection.

2. Dispositif selon la revendication 1, **caractérisé en ce que** les deux parties (16 et 18) à l'extrémité distale du toron (11) sont assemblées matériellement l'une à l'autre et une des deux parties (16 ou 18) présente un prolongement s'étendant au-delà de la pointe (17) du toron (11) et qui forme un autre tranchant.

3. Dispositif selon une des revendications 1 ou 2, **caractérisé en ce que** la partie (16) du toron (11) présentant un plus petit rayon de courbure, qui est de forme convexe, se prolonge par un rayon de sens contraire ou par une partie droite (19) en une pointe (17) du couteau et par un rayon de sens contraire dans la partie reliée au fil d'actionnement.

4. Dispositif selon la revendication 1 ou 2, **caractérisé en ce que** le tube (1) peut être électrisé à son extrémité distale en vue de pénétrer dans la muqueuse.

5. Dispositif selon la revendication 1 ou 2, **caractérisé en ce que** l'outil à haute fréquence (6) est relié à un dispositif de signalisation tactile (23), qui transmet l'atteinte des positions fonctionnelles sous forme de signal tactile à l'élément d'actionnement (2).

6. Dispositif selon la revendication 5, **caractérisé en ce que** le dispositif de signalisation tactile (23) est formé par une douille de liaison (13) fixant l'un à l'autre le fil d'actionnement (10) et la première extrémité du toron (11), une douille d'entraînement (21) fixée à l'extrémité libre (20) du toron (11), par rapport à laquelle l'autre partie du toron (11) conduisant au fil d'actionnement (10) est librement mobile, et une douille (22) fixée à l'extrémité distale de la lumière (9) dans le tube (1), et qui agit comme butée axiale pour la douille d'entraînement (21).

7. Dispositif selon la revendication 1 ou 2, **caractérisé en ce que**, dans la position de travail du dispositif, dans laquelle il forme le couteau en forme de croissant, le toron (11) s'applique avec sa première extrémité, à partir de la partie courbée en sens contraire, en décalage par rapport à l'axe central longitudinal, sur l'intérieur de la douille (22) et sur l'intérieur de la douille d'entraînement (21) et se prolonge ensuite par un coude (14) en un tracé concentrique.

8. Dispositif selon la revendication 1 ou 2, **caractérisé en ce que** l'outil à haute fréquence (6) peut tourner dans les positions du fil d'actionnement (10) avec un contour extérieur en forme de croissant et avec un contour extérieur en forme d'épingle à cheveux du toron (11).

9. Dispositif selon une des revendications 1 ou 2, **caractérisé en ce que** le fil d'actionnement (10) est fabriqué en Nitinol.

10. Dispositif selon l'une quelconque des revendications 2 à 9, **caractérisé en ce que** les deux parties (16 et 18) sont fixées l'une à l'autre à l'extrémité distale du toron (11) par soudage au laser.

11. Dispositif selon l'une quelconque des revendications 2 à 10, **caractérisé en ce que** les deux parties (16 et 18) sont fabriquées en des matériaux différents.

12. Dispositif selon l'une quelconque des revendications 2 à 11, **caractérisé en ce que** les deux parties (16 et 18) sont réalisées avec des sections transversales et/ou des formes de section transversale différentes.

13. Dispositif selon l'une quelconque des revendications 2 à 12, **caractérisé en ce que** le dispositif destiné à l'incision circulaire de la muqueuse occupe une autre position, dans laquelle le toron (11) est tiré dans la lumière (9) à un point tel que seul le prolongement en aiguille (25) soit encore saillant au-delà de l'extrémité distale du tube (1).

14. Dispositif selon l'une quelconque des revendications 2 à 13, **caractérisé en ce que** le prolongement présente un tracé rectiligne.

15. Dispositif selon l'une quelconque des revendications 2 à 13, **caractérisé en ce que** le prolongement présente un tracé courbe.
